# EUROPÄISCHE PATENTSCHRIFT

(11) **EP 1 740 569 B1**
(45) Veröffentlichungstag und Bekanntmachung des Hinweises auf die Patenterteilung: **20.02.2008**
(21) Anmeldenummer: 05731221.7
(22) Anmeldetag: 07.04.2005
(51) Int. Cl.: C07D 401/06, C07D 401/14, C07D 409/14, C07D 417/06, A61K 31/435, A61K 31/41, A61P 7/00

(54) **IMIDAZOL-DERIVATE ALS TAFIA-INHIBITOREN**
IMIDAZOLE DERIVATIVES USED AS TAFIA INHIBITORS
DERIVES D'IMIDAZOLE SERVANT D'INHIBITEURS DE TAFIA

(30) Priorität: 22.04.2004 DE 102004020186
(43) Veröffentlichungstag der Anmeldung: 10.01.2007
(60) Teilanmeldung: 07017340.6
(73) Patentinhaber: Sanofi-Aventis Deutschland GmbH, 65929 Frankfurt am Main (DE)
(72) Erfinder: KALLUS, Christopher, 65929 Frankfurt (DE); HEITSCH, Holger, 55252 Mainz-Kastel (DE); LINDENSCHMIDT, Andreas, 65812 Bad Soden (DE); GRUENEBERG, Sven, 65779 Kelkheim (DE); SZILLAT, Hauke, 65931 Frankfurt am Main (DE)
(86) Internationale Anmeldenummer: PCT/EP2005/003630
(87) Internationale Veröffentlichungsnummer: WO 2005/105781

(56) Entgegenhaltungen:
- WO-A-03/013526
- BARROW ET AL.: "Synthesis and Evaluation of Imidazole Acetic Acid Inhibitors of Activated Thrombin-Activatable Fibrinolysis Inhibitor as Novel Antithrombotics" J. MED. CHEM., Bd. 46, 2003, Seiten 5294-5297, XP002338234
- NANTERMET ET AL.: "Imidazole acetic acid TAFAIa inhibitors: SAR studies centered around the basic P'1 group" BIOORG. & MED. CHEM. LETT., Bd. 14, September 2004 (2004-09), Seiten 2141-2145, XP002338235

## Beschreibung

Die Erfindung betrifft neue Verbindungen der Formel la, die das Enzym TAFIa (aktivierter Thrombin-aktivierbarer Fibrinolyse Inhibitor) inhibieren, Verfahren zu ihrer Herstellung und Verwendung derselben als Arzneimittel.

Das Enzym TAFIa entsteht beispielsweise durch Thrombinaktivierung aus dem Thrombin-aktivierbaren-Fibrinolyse-Inhibitor-Zymogen (TAFI). Das Enzym TAFI wird auch als Plasma Procarboxypeptidase B, Procarboxypeptidase U oder als Procarboxypeptidase R bezeichnet und ist ein Carboxypeptidase B ähnliches Proenzym (L. Bajzar, Arterioscler. Thromb. Vasc. Biol. 2000, Seiten 2511 - 2518).

Während einer Gerinnselbildung wird Thrombin als das Endprodukt der Koagulationskaskade generiert und induziert die Konversion von löslichem Plasmafibrinogen zu einer unlöslichen Fibrinmatrix. Gleichzeitig aktiviert Thrombin den endogenen Fibrinolyse-Inhibitor TAFI. Aktiviertes TAFI (TAFla) entsteht also während der Thrombusbildung und der Lyse aus dem Zymogen TAFI unter der Einwirkung von Thrombin, Thrombomodulin im Komplex mit Thrombin verstärkt diesen Effekt etwa 1250 fach. TAFIa spaltet basische Aminosäuren am carboxy-Ende der Fibrinfragmente. Der Verlust der carboxy-terminalen Lysine als Bindestellen für Plasminogen führt dann zu einer Inhibition der Fibrinolyse. Effektive Inhibitoren von TAFIa verhindern den Verlust dieser hoch affinen Lysin-Bindungsstellen für Plasminogen und unterstützen auf diese Weise die endogene Fibrinolyse durch Plasmin: TAFIa Inhibitoren wirken profibrinolytisch.

Um die Hämostase im Blut aufrechtzuerhalten, haben sich Mechanismen ausgebildet, die zur Blutgerinnung und zur Auflösung von Gerinnseln führen; diese stehen in einem Gleichgewicht. Wenn ein gestörtes Gleichgewicht die Koagulation begünstigt, entsteht Fibrin in größeren Mengen, so dass pathologische Vorgänge der Thrombusbildung zu schweren Krankheitsbildern im Menschen führen können.
Genauso wie eine überschießende Koagulation zu schwerwiegenden thrombotisch bedingten Krankheitsbildern führen kann, besitzt eine antithrombotische Behandlung das Risiko von nicht erwünschten Blutungen durch eine Störung der Bildung eines notwendigen hämostatischen Pfropfs. Die Hemmung von TAFIa verstärkt - ohne die Koagulation und die Plättchenaggregation zu beeinflussen - die endogene Fibrinolyse d.h. das gestörte Gleichgewicht wird zugunsten der Fibrinolyse verschoben. So kann sowohl dem Aufbau eines klinisch relevanten Thrombus entgegengewirkt, als auch die Lyse eines schon bestehenden Gerinnsels verstärkt werden. Andererseits wird der Aufbau eines hämostatischen Pfropfs nicht beeinträchtigt, sodass eine Blutungsdiathese eher nicht zu erwarten ist (Bouma et al., J. Thrombosis and Haemostasis, 1, 2003, Seiten 1566 - 1574).

Inhibitoren von TAFIa sind bereits in den Internationalen Anmeldungen WO03/013526 und WO03/061653 beschrieben worden.

Die erfindungsgemäßen TAFIa Inhibitoren eignen sich für eine prophylaktische als auch für eine therapeutische Anwendung am Menschen, die an Erkrankungen leiden, die mit Thrombosen, Embolien, Hyperkoagulabilität oder fibrotischen Veränderungen einhergehen. Sie eignen sich sowohl für eine akute als auch für eine Langzeittherapie.

Die Erfindung betrifft daher eine Verbindung der Formel la 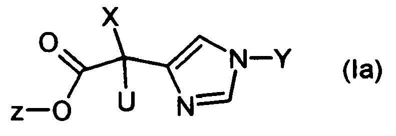 und/oder alle stereoisomeren Formen der Verbindung der Formel la und/oder Gemische dieser Formen in jedem Verhältnis, und/oder ein physiologisch verträgliches Salz der Verbindung der Formel la, wobei
U für Wasserstoffatom steht,
X für den Rest der Formel II

   -(A1)ₘ-A2 (II)

   steht, worin
   m die ganze Zahl 1 bedeutet,
   A1 für -CH₂- steht,
   A2 für Aminopyridyl steht, worin Aminopyridyl unsubstituiert oder unabhängig voneinander ein-, zwei- oder dreifach durch Halogen oder -CH₃ substituiert ist,
Y für -(C₃-C₈)-Cycloalkyl steht, worin Cycloalkyl unsubstituiert oder unabhängig voneinander ein-, zwei- oder dreifach durch R1 substituiert ist, wobei R1 für
   a) Phenyl, wobei Phenyl unsubstituiert oder ein-, zwei- oder dreifach unabhängig voneinander durch -(C₁-C₄)-Alkyl substituiert ist,
   b) Triazolyl oder Pyridinyl,
   c) -(C₁-C₄)-Alkyl,
   d) -(C₃-C₆)-Cycloalkyl,
   e) -CF₃,
   f) -O-CF₃,
   g) Fluor oder
   h) Chlor steht, und
Z für 1) Wasserstoffatom,
   2) -(C₁-C₆)-Alkyl,
   3) -(C₁-C₆)-Alkyl-OH,
   4) -(C₀-C₄)-Alkyl-(C₃-C₆)-Cycloalkyl oder
   5) -(C₁-C₁₀)-Alkyl-O-C(O)-O-(C₃-C₆)-Cycloalkyl steht.

Ein weiterer Gegenstand der Erfindung ist die Verbindung der Formel la, wobei
U für Wasserstoffatom steht,
X für den Rest der Formel II steht, worin
   m die ganze Zahl 1 bedeutet,
   A1 für -CH₂- steht,
   A2 für den Rest 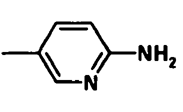 steht, welcher unsubstituiert oder unabhängig voneinander ein-, zwei- oder dreifach durch F, Cl, Br, J oder -CH₃ substituiert ist,
Y für -(C₃-C₈)-Cycloalkyl steht, worin Cycloalkyl unsubstituiert oder unabhängig voneinander ein-, zwei- oder dreifach durch R1 substituiert ist, wobei R1 für
   a) Phenyl, wobei Phenyl unsubstituiert oder ein-, zwei- oder dreifach unabhängig voneinander durch -(C₁-C₄)-Alkyl substituiert ist,
   b) Pyridyl oder Tetrazolyl,
   c) -(C₁-C₄)-Alkyl,
   d) -(C₃-C₆)-Cycloalkyl,
   e) -CF₃,
   f) -O-CF₃,
   g) Fluor oder
   h) Chlor steht, und
Z für Wasserstoffatom steht.

Ein weiterer Gegenstand der Erfindung sind Verbindungen der Formel la aus der Reihe
3-(6-Amino-pyridin-3-yl)-2-(1-cyclohexyl-1H-imidazol-4-yl)-propionsäure,
3-(6-Amino-pyridin-3-yl)-2-(1-cyclohexyl-1H-imidazol-4-yl)-propionsäure-methylester,
3-(6-Amino-pyridin-3-yl)-2-(1-cyclohexyl-1H-imidazol-4-yl)-propionsäure-isopropylester,
3-(6-Amino-pyridin-3-yl)-2-(1-cyclohexyl-1H-imidazol-4-yl)-propionsäure-cyclopropylmethylester,
3-(6-Amino-pyridin-3-yl)-2-(1-cyclohexyl-1H-imidazol-4-yl)-propionsäure-2-hydroxy-ethylester,
3-(6-Amino-pyridin-3-yl)-2-(1 -cyclohexyl-1H-imidazol-4-yl)-propionsäure-1-cyclohexyloxycarbonyloxy-ethylester,
3-(6-Amino-pyridin-3-yl)-2-(1-cyclopentyl-1H-imidazol-4-yl)-propionsäure,
3-(6-Amino-pyridin-3-yl)-2-[1-(4,4-dimethyl-cyclohexyl)-1H-imidazol-4-yl]-propionsäure, oder
3-(6-Amino-pyridin-3-yl)-2-(1-cyclohexyl-1H-imidazol-4-yl)-propionsäureethylester.

Unter dem Begriff "(C₁-C₆)-Alkyl" oder "(C₁-C₁₀)-Alkyl" werden Kohlenwasserstoffreste verstanden, deren Kohlenstoffkette geradkettig oder verzweigt ist und 1 bis 6 Kohlenstoffatome oder 1 bis 10 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan, Neohexyl, Heptyl, Octanyl, Nonanyl oder Decanyl.

Unter dem Begriff "(C₀-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "-C₀-Alkylen" ist eine kovalente Bindung.

Unter dem Begriff "(C₃-C₈)-Cycloalkyl" werden Reste verstanden wie Verbindungen, die sich von 3- bis 8-gliedrige Monocyclen wie Cyclopropyl, Cyclobutyl, Cyclopentyl, Cyclohexyl, Cycloheptyl oder Cyclooctanyl herleiten.

Die erfindungsgemäßen Verbindungen können nach wohlbekannten Verfahren oder nach hier beschriebenen Verfahren hergestellt werden.

Funktionelle Gruppen der verwendeten Intermediate, beispielsweise Amino- oder Carboxylgruppen können dabei durch geeignete Schutzgruppen maskiert werden. Geeignete Schutzgruppen für Aminofunktionen sind beispielweise die t-Butoxycarbonyl-, die Benzyloxycarbonyl- oder die Phtalolylgruppe sowie die Trityl- oder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Green, T.W., Wutz, P.G.M., Protective Groups in Organic Synthesis (1991), 2nd Ed., Wiley-Interscience, oder Kocienski, P., Protecting Groups (1994), Thieme).

Der Begriff Schutzgruppe kann auch entsprechende polymergebundene Schutzgruppen umfassen. Solcherart maskierte Verbindungen gemäß Formel (Ia), in der die funktionellen Gruppen des Restes X gegebenenfalls ebenfalls maskiert sein können, können, obwohl gegebenenfalls selber pharmakologisch nicht aktiv, gegebenenfalls nach Administration in Säugern durch Metabolisierung zu den erfindungsgemäßen, pharmakologisch aktiven Verbindungen umgewandelt werden.

Die Erfindung betrifft ferner ein Verfahren zur Herstellung der Verbindung der Formel la und/oder einer stereoisomeren Form der Verbindung der Formel la und/oder eines physiologisch verträglichen Salzes der Verbindung der Formel la, das dadurch gekennzeichnet ist, dass man
a) die Verbindung der Formel la nach Schema 1 herstellt, wobei X und Y die jeweils oben angegebenen Bedeutungen haben:

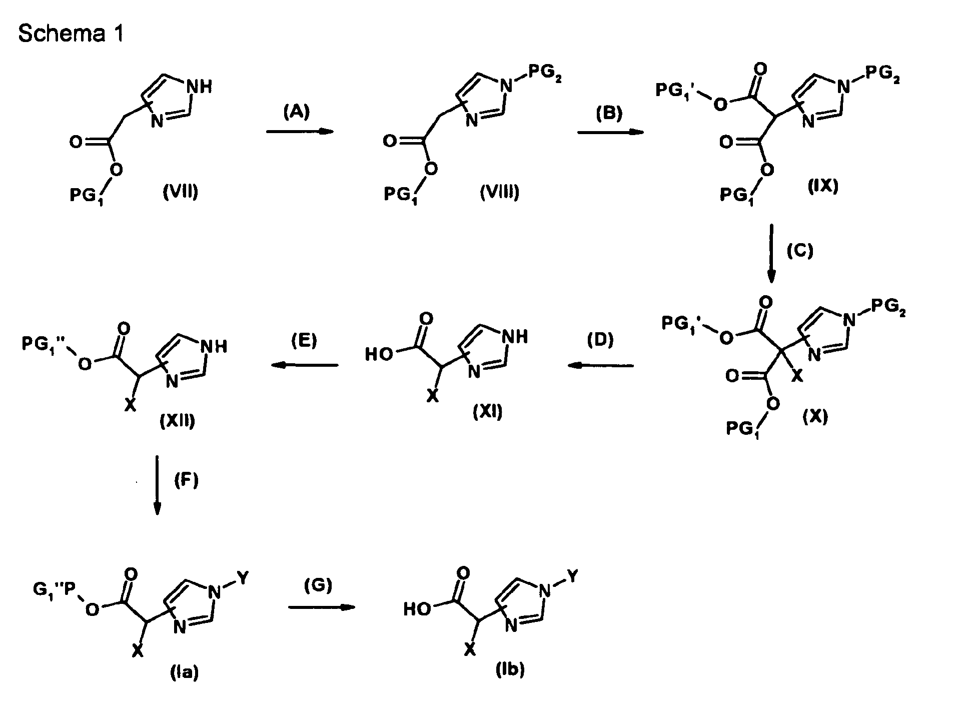

Die Verbindungen gemäß (VII) können nach üblichen Methoden, beispielsweise aus 4-Imidazolessigsäure-Hydrochlorid durch Umsetzung in niederen Alkoholen in Gegenwart von Thionylchlorid erhalten werden, wobei PG1 für eine geeignete Carboxylschutzgruppe steht.
In einem Verfahrensschritt (A) wird nach Standardverfahren eine geeignete Schutzgruppe PG2 eingeführt.
Die resultierenden Verbindungen (VIII) werden im Verfahrensschritt (B) in Gegenwart einer Base in einem inerten Lösungsmittel bei Temperaturen zwischen -90°C und 50 °C mit einer Verbindung der Formel 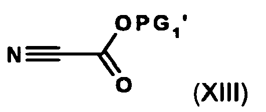 zu den Verbindungen (IX) umgesetzt, wobei PG1' für eine geeignete Carboxylschutzgruppe steht.
Aus den Verbindungen (IX) erhält man im Verfahrensschritt (C) in Gegenwart einer starken Base in einem inerten Lösungsmittel bei Temperaturen zwischen -90 °C und + 50 °C durch Umsetzung mit Verbindungen der Formel

**X-LG** (XIV)

die Verbindungen (X), wobei in X enthaltene funktionelle Gruppen durch geeignete Schutzgruppen maskiert sein können und LG eine geeignete aktivierende Gruppe wie beispielsweise Chlor, Brom, lod, Mesylat, Tosylat oder Triflat darstellt.
Im Verfahrensschritt (D) werden die Verbindungen (X) zu den Verbindungen (XI) umgesetzt, indem die Schutzgruppen PG1, PG1' und PG2 sowie gegebenenfalls die in X enthaltene Schutzgruppe nach Standardverfahren entfernt werden und gegebenenfalls unter wäßrig-sauren Bedingungen bei Temperaturen zwischen Raumtemperatur und 100 °C behandelt wird.
Die Verbindungen (XII) können aus den Verbindungen (XI) in einem Schritt (E) erhalten werden, indem unter Standardbedingungen eine geeignete Carboxylschutzgruppe PG1" eingeführt wird.

In einem Schritt (F) können die Verbindungen gemäß (la) erhalten werden, indem die Verbindungen (XII) in Gegenwart einer Base bei Temperaturen zwischen -90°C und +60 °C in einem inerten Lösungsmittel mit Verbindungen der Formel

**Y-LG** (XV),

wobei LG eine geeignete aktivierende Gruppe wie Chlor, Brom, lod, Mesylat, Tosylat oder Triflat darstellt und Y die oben angegebenen Bedeutungen hat, umgesetzt werden.
Alternativ können die Verbindungen (la) erhalten werden, indem die Verbindungen (XII) unter Mitsonobu-Bedingungen mit Verbindungen der Formel

**Y-OH** (XVI),

in denen Y die oben angegebenen Bedeutungen hat, umgesetzt werden.
Alternativ können die Verbindungen (la) erhalten werden, indem die Verbindungen (XII) in Gegenwart einer Base bei Temperaturen zwischen -90°C und +60 °C in einem inerten Lösungsmittel mit sechsgliedrigen 2-Fluronitroaromaten oder sechsgliedrigen 4-Fluronitroaromaten umgesetzt werden. Anschließend wird die Nitrogruppe nach Standardverfahren, z. B. bei Raumtemperatur in niederen Alkoholen mit Wasserstoff in Gegenwart eines Übergangsmetallkatalysators oder in inerten Lösungsmitteln in Gegenwart von Zinn(II)chlorid-Dihydrat zur Aminogruppe reduziert und nach Standardverfahren acyliert.
Die Verbindungen gemäß (Ib) werden im Schritt (G) erhalten, in dem die Schutzgruppe PG1" und gegebenenfalls die in X enthaltene Schutzgruppe unter Standardbedingungen entfernt wird.
Die Verbindungen (XIII), (XIV), (XV) und (XVI) sind kommerziell erhältlich, literaturbekannt oder können nach literaturbekannten Verfahren hergestellt werden.

Die Umsetzungen können bei normalem, erhöhtem oder bei erniedrigtem Druck durchgeführt werden. Im Allgemeinen arbeitet man bei Normaldruck.
Als Lösungsmittel für die Verfahrensschritte (B), (C) und (F) eignen sich inerte organische Lösungsmittel. Hierzu gehören beispielsweise Ether wie Dioxan, THF oder 1,2-Dimethoxyethan, Kohlenwasserstoffe wie Cyclohexan, Benzol, Toluol oder Xylol, Nitroaromaten wie Nitrobenzol, Carbonsäureamide wie Dimethylformamid oder Dimethylacetamid, Alkylsulfoxide wie Dimethylsulfoxid, aliphatische Nitrile wie Acetonitril, oder andere Lösungsmittel wie N-Methylpyrrolidinon. Ebenso ist es möglich, Gemische der genannten Lösungsmittel anzuwenden.
Als Basen für die die Verfahrensschritte (B), (C) und (F) eignen sich die üblichen anorganischen und organischen Basen. Hierzu gehören bevorzugt Alkali- und Erdalkalicarbonate wie Natrium-, Kalium oder Calciumcarbonat, Alkalihydride wie Natriumhydrid, Amide wie Lithium-bis(trimethylsilyl)amid oder Lithium-düsopropylamid, organische Amine wie Pyridin, 4-N,N-Dimethylaminopyridin, Triethylamin, Ethyldiisopropylamin, N-Methylmorpholin, N-Methylpiperidin, 1,5-Diazabicyclo(4.3.0)non-5-en (DBN) oder 1,8-Diazabicyclo(5.4.0)undec-7-en (DBU), oder metallorganische Verbindungen wie Butyllithium oder Phenyllithium. Besonders bevorzugt sind Natriumhydrid, Lithium-bis(trimethylsilyl)amid und Triethylamin.
Unter Mitsonobu-Bedingungen ist im Allgemeinen die Verwendung von inerten Lösungsmitteln in Gegenwart eines Azodicarboxylats, gegebenenfalls in Gegenwart eines Zusatzreagenzes, bevorzugt in einem Temperaturbereich von 0 °C bis Raumtemperatur bei Normaldruck gemeint. Inerte Lösungsmittel sind beispielsweise Halogenkohlenwasserstoffe wie Methylenchlorid, Ether wie Dioxan, THF oder 1,2-Dimethoxyethan, Kohlenwasserstoffe wie Benzol, Toluol oder Xylol, Nitroaromaten wie Nitrobenzol, Carbonsäureamide wie Dimethylformamid oder Dimethylacetamid, Alkylsulfoxide wie Dimethylsulfoxid, aliphatische Nitrile wie Acetonitril, Ester wie Benzoesäureethylester oder andere Lösungsmittel wie N-Methylpyrrolidinon. Ebenso ist es möglich, Gemische der genannten Lösungsmittel anzuwenden.
Übliche Zusatzreagenzien für die Mitsonobu-Reaktion sind beispielweise Triphenylphosphin, Diphenyl-(2-pyridyl)-phosphin oder (4-Dimethylaminophenyl)-diphenylphosphin.
Azodicarboxylate sind beispielsweise Diethylazodicarboxylat, Dimethylazodicarboxylat, Düsopropylazodicarboxylat oder Di-tert-butylazodicarboxylat.

Eine nach Schema 1 hergestellte Verbindung der Formel la, oder eine geeignete Vorstufe der Formel la, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt (Verfahren b), oder
die nach Schema 1 hergestellte Verbindung der Formel Ia entweder in freier Form isoliert oder im Falle des Vorliegens von sauren oder basischen Gruppen in physiologisch verträgliche Salze umwandelt (Verfahren c).

Im Verfahrensschritt b) wird die Verbindung der Formel la, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel la zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel la, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenrreine Ausgangsprodukte einzusetzen. Dadurch können ggf. auch andere oder vereinfachte Verfahren zur Aufreinigung der Endprodukte eingesetzt werden. Diese Ausgangsprodukte wurden zuvor nach Literatur-bekannten Verfahren enantiomeren- oder diastereomerenrein hergestellt. Das kann insbesondere bedeuten, daß in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Saure oder basische Produkte der Verbindung der Formel la können in Form ihrer Salze oder in freier Form vorliegen. Bevorzugt sind pharmakologisch verträgliche Salze, beispielsweise Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.
Die Herstellung physiologisch verträglicher Salze aus zur Salzbildung befähigten Verbindungen der Formel la, einschließlich deren stereoisomeren Formen, gemäß Verfahrensschritt c) erfolgt in an sich bekannter Weise. Die Verbindungen der Formel la bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze Sofern die Verbindungen der Formel Ia basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Die Erfindung betrifft auch Arzneimittel, gekennzeichnet durch einen wirksamen Gehalt an mindestens einer Verbindung der Formel la und/oder eines physiologisch verträglichen Salzes der Verbindung der Formel la und/oder eine gegebenenfalls stereoisomere Form der Verbindung der Formel la, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, die durch eine Hemmung von TAFla behandelbar sind. So eignen sich TAFla Inhibitoren sowohl für eine prophylaktische als auch für eine therapeutische Anwendung am Menschen. Sie eignen sich sowohl für eine akute Behandlung als auch für eine Langzeittherapie. TAFIa Inhibitoren können eingesetzt werden in Patienten, die an Störungen des Wohlbefindens oder Krankheiten leiden, die mit Thrombosen, Embolien, Hyperkoagulabilität oder fibrotischen Veränderungen einhergehen.
Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können TAFIa Inhibitoren eingesetzt werden bei allen Eingriffen, die zu einem Kontakt des Blutes mit Fremdoberflächen führen wie z. B. bei Dialysepatienten und Patienten mit Verweilkathetern. TAFIa Inhibitoren können eingesetzt werden um die Thrombosegefahr nach chirurgischen Eingriffen wie bei Knie- und Hüftgelenksoperationen zu reduzieren.

TAFIa Inhibitoren eignen für die Behandlung von Patienten mit disseminierter intravaskulärer Koagulation, Sepsis und anderen intravaskulären Ereignissen, die mit einer Entzündung einhergehen. Weiterhin eignen sich TAFIa Inhibitoren für die Prophylaxe und Behandlung von Patienten mit Atherosklerose, Diabetes und dem metabolischen Syndrom und dessen Folgen. Störungen des hämostatischen Systems (z.B. Fibrinablagerungen) wurden impliziert in Mechanismen, die zu Tumorwachstum und Tumormetastasierung führen; TAFla Inhibitoren eignen sich zur Verlangsamung oder Verhinderung solcher Prozesse.

Weitere Indikationen für den Einsatz von TAFla Inhibitoren sind fibrotische Veränderungen der Lunge wie die chronische obstruktive Lungenerkrankung, das adult respiratory distress syndrome (ARDS) und des Auges wie Fibrinablagerungen nach Augenoperationen. TAFIa Inhibitoren eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung mit TAFIa Inhibitoren von Stents und anderen Oberflächen, die im Körper mit Blut in Kontakt kommen, ist möglich.

Die Erfindung betrifft auch ein Verfahren zur Herstellung eines Arzneimittels, das dadurch gekennzeichnet, dass man mindestens eine Verbindung der Formel la mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen. Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel Ia enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel la, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

TAFIa Inhibitoren können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden.

### Beispiele

Endprodukte werden in der Regel durch massenspektroskopische Methoden (FAB-, ESI-MS) und ¹H-NMR bestimmt, angegeben sind jeweils der Hauptpeak oder die beiden Hauptpeaks. Temperaturangaben in Grad Celsius, RT bedeutet Raumtemperatur (21 °C bis 24 °C). Verwendete Abkürzungen sind entweder erläutert oder entsprechen den üblichen Konventionen.
Wenn nicht anders aufgeführt, wurden die LC/MS-Analysen unter folgenden Bedingungen durchgeführt:
Methode A: Säule: YMC Jsphere 33x2.1 mm, Packungsmaterial 4 µm, Laufmittel: CH₃CN + 0.05% Trifluoressigsäure (TFA) : H₂O + 0.05% TFA, Gradient: 5:95 (0 min.) nach 95:5 (3.4 min.), Fluß: 1 mL/min., Temperatur: 30 °C;
Methode B: Säule: YMC Jsphere ODS H80 20x2.1 mm, Packungsmaterial 4 µm, Laufmittel: CH₃CN + 0.05% Trifluoressigsäure (TFA): H₂O + 0.05% TFA, Gradient: 4:96 (0 min.) nach 95:5 (2.0 min.), Fluß: 1 mL/min., Temperatur: 30 °C;
Methode C: Säule: YMC Jsphere 33x2.1 mm, Packungsmaterial 4 µm, Laufmittel: CH₃CN + 0.05% Trifluoressigsäure (TFA) : H₂O + 0.05% TFA, Gradient: 5:95 (0 min.) nach 95:5 (2.5 min.), Fluß: 1.3 mL/min., Temperatur: 30 °C.
Soweit nicht anders angegeben, wurden chromatographische Trennungen an Kieselgel mit Ethylacetat/Heptan-Gemischen als Laufmittel sowie präparative Trennungen an Reversed Phase-(RP)-Kieselgel (HPLC) mit trifluoressigsäurehaltigen Wasser-Acetonitril-Gemischen als Laufmittel durchgeführt.
Das Abdampfen von Lösungsmitteln geschah in der Regel unter vermindertem Druck bei 35 °C bis 45 °C.

### Beispiel 1

### 3-(6-Amino-pyridin-3-yl)-2-(1-cyclohexyl-1H-imidazol-4-yl)-propionsäure

### Beispiel 1a

### (5-Methyl-pyridin-2-yl)-carbaminsäure-tert-butylester

Zu einer Lösung von 30,00 g (277 mmol) 5-Methyl-pyridin-2-ylamine und 3,39g (28 mmol) 4-Dimethylaminopyridin in 150 mL CH₂Cl₂ wurde eine Lösung von 60,54 g (277 mmol) Di-tert-butyldicarbonat in 50 mL CH₂Cl₂ getropft. Die resultierende Lösung wurde 16 Stunden (h) bei Raumtemperatur gerührt und anschließend zur Trockene eingeengt. Die chromatographische Reinigung an Kieselgel lieferte 15,4g des Produkts als farblosen Feststoff.
MS (ES+) = 209 [M+H]⁺

### Beispiel 1b

Eine Lösung von 14,9g (72 mmol) der Verbindung aus Beispiel 1a wurde in 700 mL CCl₄ vorgelegt und zum Sieden erhitzt. Nach Zugabe eines Gemisches aus 12,8g (72 mmol) N-Bromsuccinimid und 1,2g (7 mmol) 2,2'-Azo-bis-(isobuttersäurenitril) wurde 2,5h unter Rückfluß erhitzt. Das Reaktionsgemisch wurde heiß filtriert, der Filterrückstand wurde mit CCl₄ nachgewaschen und die vereinigten Filtrate vom Lösungsmittel befreit. Der Rückstand wurde aus Acetonitril umkristallisiert, abgesaugt, mit Acetonitril und Acetonitril/Methyl-tert-butylether (1:1) gewaschen und unter verminderten Druck getrocknet. Es wurden 6,94 g der gewünschten Verbindung in Form eines cremefarbenen Feststoffs erhalten.
¹H-NMR (500 MHz, DMSO-d₆): δ =1.48 (s, 9H), 4.71 (s, 2H), 7.78 (d, 1H), 7.82 (d, 1H), 8.32 (s, 1H).

### Beispiel 1c

### 4-Imidazol-essigsäure-methylester Hydrochlorid

5,0 g (30,75 mmol) 4-Imidazol-essigsäure wurden in 50 mL Methanol gelöst und anschließend mit 5,6 ml (76,87 mmol) Thionylchlorid versetzt. Die resultierende Lösung wurde 4h am Rückfluß erhitzt und nach dem Abkühlen zur Trockene eingeengt. Die Trocknung unter verminderten Druck ergab 5,3 g des gewünschten Produkts in Form eines blassgelben Feststoffs.
MS (ES+) = 141 [M+H]⁺

### Beispiel 1d

### [1-(Tolyl-4-sulfonyl)-1H-imidazol-4-yl]-essigsäure-methylester

Eine Lösung aus 5,0 g (28,31 mmol) der Verbindung aus Beispiel 1c und 9,8 mL Triethylamin (70,72 mmol) in 350, mL CH₂Cl₂ wurde nach Abkühlung auf 0 °C mit 7,04 g (36,90 mmol) p-Toluolsulfonylchlorid versetzt. Die Lösung wurde 15 Minuten (min) bei 0 °C und 15 min bei Raumtemperatur (RT) gerührt, eingeengt und anschließend mit einer Ammoniumchlorid-Lösung und Wasser gewaschen. Die organische Phase wurde über Na₂SO₄ getrocknet und nach Filtration zur Trockene eingeengt.
Chromatographische Reinigung an Kieselgel ergab 7,2 g des gewünschten Produkts.
Rₜ (Methode A) = 1,71 min MS (ES+) = 295 [M+H]⁺

### Beispiel 1e

### 2-[1-(Tolyl-4-sulfonyl)-1H-imidazol-4-yl)-malonsäure-dimethylester

Zu einer auf 0 °C gekühlte Lösung aus 3,0 g (10,19 mmol) der Verbindung aus Beispiel 1d in 50 mL absolutem Tetrahydrofuran (THF) wurde langsam 9,3 mL (11,1 mmol) einer 20%ige Lösung von Lithium-bis-(trimethylsilyl)-amid in THF hinzugetropft. Nach 30 min Rühren bei 0 °C wurde 0,89 mL Methylcyanoformiate hinzugefügt und die resultierende Lösung über einen Zeitraum von 1,5 h langsam auf RT erwärmt. Die Reaktionslösung wurde anschließend auf etwa 300 mL einer gesättigten Ammoniumchlorid-Lösung gegossen. Es wurde mehrfach mit Ethylacetat (EA) extrahiert und die vereinigten EA-Extrakte nach Waschen mit Wasser und Trocknung über Na₂SO₄ zur Trockene eingeengt. Chromatographische Reinigung an Kieselgel lieferte 2,2 g der Titelverbindung.
Rₜ (Methode A) = 1,89 min MS (ES+) = 353 [M+H]⁺

### Beispiel 1f

### 2-(6-tert.-Butyloxycarbonylamino-pyridin-3-yl-methyl)-2-[1-(tolyl-4-sulfonyl-1H-imidazol-4-yl]-malonsäure-dimethylester

Eine auf 0 °C gekühlte Lösung aus 2,2 g (6,24 mmol) der Verbindung aus Beispiel 1 e in 40 mL absolutem N,N'-Dimethylformamid (DMF) wurde mit 150 mg (6,26 mmol) NaH (50%ig) versetzt und 1 h bei RT gerührt. Nach Abkühlung auf 0 °C wurde 1,8 g (6,24 mmol) 2-(6-tert.-Butyloxycarbonyl-amino-pyridin-3-yl)-methylbromid hinzugefügt und die resultierende Lösung für 30 min bei 0 °C gerührt. Anschließend wurde 50 mL Wasser hinzugefügt und mehrfach mit EA extrahiert. Die vereinten EA-Extrakte wurden über Na₂SO₄ getrocknet, filtriert und zur Trockene eingeengt. Die chromatographische Reinigung des Rückstandes an Kieselgel lieferte 2,9 g der gewünschten Verbindung.
Rₜ (Methode A) = 2,10 min MS (ES+) = 559 [M+H]⁺

### Beispiel 1g

### 3-(6-Amino-pyridin-3-yl)-2-(1H-imidazol-4-yl)-propionsäure-ethylester

400,0 mg (0,72 mmol) der Verbindung aus Beispiel 1f wurde in einer Lösung aus 5 mL 37%iger Salzsäure und 5 mL Wasser suspendiert. Die erhaltene Suspension wurde für für 20 min bei 180 °C in der Mikrowelle erhitzt. Anschließend wurde die Reaktionslösung eingeengt, der erhaltene Rückstand in 50 mL Ethanol aufgenommen und erneut eingeengt. Der verbliebene Rückstand wurde in 50 mL Ethanol gelöst und die Lösung nach Zusatz von 40 mL einer mit gasförmigen HCl gesättigten Ether-Lösung für 3 h gerührt. Es wurde zur Trockene eingeengt, der Rückstand in 20 mL einer Mischung aus EA und einer gesättigten NaHCO₃-Lösung aufgenommen und die Reaktionslösung mehrfach mit EA extrahiert. Die vereinten EA-Extrakte wurden über Na₂SO₄ getrocknet, filtriert und eingeengt. Die chromatographische Reinigung des Rückstandes an Kieselgel lieferte 134 mg der gewünschten Verbindung.
Rₜ (Methode A) = 0,20 min MS (ES+) = 261 [M+H]⁺

### Beispiel 1h

### 3-(6-Amino-pyridin-3-yl)-2-(1-cyclohexyl-1H-imidazol-4-yl)-propionsäure-ethylester Methode 1:

Eine Lösung aus 60,1 mg (0,23 mmol) der Verbindung aus Beispiel 1g, 37,7 mg (0,23 mmol) Bromocyclohexan and 100 µL Triethylamin in 1 mL absolutem THF wurde für 40 min bei 170 °C in der Mikrowelle behandelt. Anschließend wurde die Reaktionslösung in wenig EA/Wasser (1:1) aufgenommen, die Phasen abgetrennt, die organische Phase wurde über Na₂SO₄ getrocknet, filtriert und zur Trockene eingeengt. Chromatographische Reinigung an RP-Kieselgel mit CH₃CN/Wasser/0,1% TFA als Laufmittel und Gefriertrocknung der vereinten Wertfraktionen lieferte 18,0 mg der gewünschten Verbindung als Bistrifluoracetat in Form eines amorphen Feststoffs.

Alternativ zur Methode 1 wurde die Titelverbindung auch nach nachfolgend beschriebenen Methode 2 hergestellt.

### Methode 2:

Eine Lösung aus 200,0 mg (0,77 mmol) der Verbindung aus Beispiel 1e und 39,0 mg (0,77 mmol, 60%ig) NaH in 5 ml absoluten DMF wurde für 1h bei RT gerührt und anschließend mit 123,7 mg (0,77 mmol) 3-Bromcyclohexen versetzt. Die resultierende Lösung wurde 1 h bei RT gerührt. Nach Zugabe von 2 ml Wasser wurde mehrfach mit EE extrahiert und die vereinten EE-Extrakte über MgSO₄ getrocknet. Nach Einengung, chromatographischer Reinigung des Rückstandes an RP-Kieselgel mit Wasser/Acetonitril (5:95) und Einengung der Wertfraktionen resultierten 152 mg 3-(6-Amino-pyridin-3-yl)-2-(1-cyclohex-2-en-1H-imidazol-4-yl)-propionsäure-ethylester. Diese Verbindung wurde anschließen in 15 ml Methanol in Gegenwart von Pd/Aktivkohle (10%) 2 h bei RT hydriert. Nach Einengung und Trocknung unter verminderten Druck resultierten 127 mg der gewünschten Titelverbindung in Form eines amorphen Feststoffs.
Rₜ (Methode A) = 0.89 min MS (ES+) = 343 [M+H]⁺
¹H-NMR (500 MHz, DMSO-d₆): δ = 1.12 (t, 3H), 1.18 (m, 1H), 1.38 (m, 2H), 1.68, (m, 3H), 1.80 (m, 2H), 2.03 (m, 2H), 3.08 (dd, 1H), 3.15 (dd, 1H), 3.42 (q, 2H), 4.10 (dt, 1H), 4.24 (m, 1H), 6.95 (d, 1H), 7.75 (m, 3H), 8.10 (s, 2H).

### Beispiel 1i

3-(6-Amino-pyridin-3-yl)-2-(1-cyclohexyl-1H-imidazol-4-yl)-propionsäure Hydrochlorid Eine Lösung aus 13,7 mg (0,03 mmol) der Verbindung aus Beispiel 1h in 0,5 mL Wasser und 0,5 mL 37%iger Salzsäure wurde für 5 min bei 180 °C in der Mikrowelle behandelt. Anschließend wurde unter verminderten Druck zur Trockene eingeengt, der Rückstand in wenig Wasser aufgenommen und die Lösung gefriergetrocknet. Dabei resultierten 8,0 mg der Titelverbindung als Bishydrochlorid in Form eines amorphen Feststoffs.
Rₜ (Methode A) = 0.80 min MS (ES+) = 315 [M+H]⁺
¹H-NMR (400 MHz, DMSO-d₆): δ = 0,86 (m, 1H), 1,28 (m, 5H), 1,60 (m, 3H), 1,78 (m, 2H), 1,92 (m, 2H), 2,77 (dd, 1H), 2,95 (dd, 1H), 3,55 (dt, 1H), 3,95 (m, 1H), 5,60 (s, 2H), 6,34 (d, 1H), 7,00 (s, 1H), 7,14 (dd, 1H), 7,58 (s, 1H), 7,68 (s, 1H).

Die beiden Enantiomeren der Verbindung aus Beispiel 1g wurden via präparativer Chromatographie an chiraler Phase getrennt; Phase: Chiralpak ADH40, Säulenmaße: 250 x 4 mm, Laufmittel: Heptan:Ethanol:Methanol 8:1:1 plus 0.1 % Ammoniumacetat (isokratisch), Flowrate: 1 mL/min., Temperatur: 30°C:
Enantiomer 1: Rₜ = 6,13 min. Enantiomer 2: Rₜ = 46,32 min.

### Beispiel 2

3-(6-Amino-pyridin-3-yl)-2-(1-cyclohexyl-1H-imidazol-4-yl)-propionsäure-methylester Eine Lösung aus 50.0 mg (0.16 mmol) der Verbindung aus Beispiel 1i in 8 mL Methanol wurde mit 3 mL einer HCl-gesättigten Ether-Lösung versetzt und 6 h bei Raumtemperatur gerührt. Anschließend wurde die Lösung zur Trockene eingeengt und der resultierende Rückstand im Hochvakuum getrocknet. Es resultierten 51 mg der Titelverbindung als Bishydrochlorid in Form eines amorphen Feststoffs.
Rₜ (Methode A) = 0,90 min MS (ES+) = 329 [M+H]⁺
¹H-NMR (500 MHz, DMSO-d₆): δ = 1.20 (m, 1H), 1.37 (m, 2H), 1.65 (m, 3H), 1.82 (m, 2H), 2.0 (m, 2H), 3.10 (dd, 1H), 3.18 (dd, 1H), 3.65 (s, 3H), 4.22 (m, 2H), 6.95 (d, 1H), 7.72 (m, 3H), 8.05 (s, 2H)
Die beiden Enantiomeren der Verbindung wurden via präparativer Chromatographie an chiraler Phase getrennt; Phase: Chiracel OD/H-61, Laufmittel:
Heptan:Propanol:Methanol 15:1:1 plus 0,1% Diethylamin (isokratisch), Flowrate: 1 mL/min., Temperatur: 30°C:
Enantiomer 1: Rₜ = 14,05 min. Enantiomer 2: Rₜ = 17,15 min.

### Beispiel 3

3-(6-Amino-pyridin-3-yl)-2-(1-cyclohexyl-1H-imidazol-4-yl)-propionsäure-isopropylester Eine Lösung aus 38.0 mg (0.12 mmol) der Verbindung aus Beispiel 1i in 5 mL Isopropanol wurde mit 2 mL einer HCl-gesättigten Ether-Lösung versetzt und 4 h bei RT gerührt. Anschließend wurde die Lösung zur Trockene eingeengt und der resultierende Rückstand im Hochvakuum getrocknet. Es resultierten 25 mg der Titelverbindung als Bishydrochlorid in Form eines amorphen Feststoffs.
Rₜ (Methode A) = 0.92 min MS (ES+) = 357 [M+H]⁺
¹H-NMR (500 MHz, DMSO-d₆): δ = 1.02 (d, 6H), 1.15 (m, 3H), 1.38 (m, 2H), 1.68 (m, 3H), 1.85 (m, 1H), 2.03 (m, 1H), 3.05 (dd, 1H), 3.14 (dd, 1H), 3.78 (dt, 1H), 4.20 (m, 2H), 4.90 (m, 1H), 6.90 (m, 1H), 7.74 (m, 3H), 8.00 (m, 1H)

### Beispiel 4

### 3-(6-Amino-pyridin-3-yl)-2-(1-cyclohexyl-1H-imidazol-4-yl)-propionsäure-cyclopropyl-methylester

Eine Lösung aus 50.0 mg (0.16 mmol) der Verbindung aus Beispiel 1i in 2 mL Cyclopropylcarbinol wurde mit 1 mL einer HCl-gesättigten Ether-Lösung versetzt und 12 h bei Raumtemperatur gerührt. Anschließend wurde die Lösung zur Trockene eingeengt und der resultierende Rückstand im Hochvakuum getrocknet. Chromatographische Reinigung an Kieselgel mit CH₂Cl₂/Methanol als Laufmittel lieferte 29 mg der Titelverbindung in Form eines amorphen Feststoffs.
Rₜ (Methode A) = 0.91 min MS (ES+) = 369 [M+H]⁺
¹H-NMR (500 MHz, DMSO-d₆): δ = 0.18 (m, 2H), 0.42 (m, 2H), 0.98 (m, 1H), 1.18 (m, 1H), 1.34 (m, 2H), 1.60 (m, 3H), 1.75 (d, 2H), 1.94 (d, 2H), 2.90 (m, 2H), 3,70 (m, 1H), 3.78 (d, 2H), 3.97 (m, 1H), 5.70 (s, 2H), 6.34 (d, 1H), 7.05 (s, 1H), 7.13 (d, 1H), 7.55 (s, 1H9, 7.70 (s, 1H)

### Beispiel 5

### 3-(6-Amino-pyridin-3-yl)-2-(1-cyclohexyl-1H-imidazol-4-yl)-propionsäure-2-hydroxy-ethylester

Eine Lösung aus 50.0 mg (0.16 mmol) der Verbindung aus Beispiel 1 in 1 mL Ethylenglykol wurde mit 0.4 mL einer HCl-gesättigten Ether-Lösung versetzt und 1 h bei RT gerührt. Anschließend wurde die Lösung zur Trockene eingeengt, der Rückstand in ges. NaHCO₃-Lösung aufgenommen und mehrfach mit EA extrahiert. Die vereinten EA-Extrakte wurden getrocknet, filtriert und eingeengt. Der resultierende Rückstand wurde im Hochvakuum getrocknet, wobei 33 mg der Titelverbindung in Form eines hellgelben Öls gewonnen wurden.
Rₜ (Methode A) = 0,85 min MS (ES+) = 359 [M+H]⁺
¹H-NMR (500 MHz, DMSO-d₆): δ = 0.85 (m, 1H), 1.15-1.35 (m, 5H), 1.60 (m, 3H), 1.78 (d, 1H), 1.95 (m, 1H), 2.90 (m, 1H), 3.48 (m, 1H), 4.00 (m, 2H), 5.68 (s, 2H), 6.32 (d, 1H), 7.05 (s, 1H), 7.13 (d, 1H). 7.55 (s, 1H), 7.65 (d, 1H)

### Beispiel 6

### 3-(6-Amino-pyridin-3-yl)-2-(1-cyclohexyl-1H-imidazol-4-yl)-propionsäure-1-cyclohexyloxycarbonyloxy-ethylester

Eine Lösung aus 50,0 mg (0,16 mmol) der Verbindung aus Beispiel 1 und 39 mg (0,19 mmol) 1-Cyclohexyloxycarbonyloxy-1-ethylchlorid, 13 mg (0.08 mmol) Kl und 26 mg (0.19 mmol) K₂CO₃ in 2 mL DMF wurde 12 h bei 60 °C gerührt. Anschließend wurde mit 5 mL Wasser versetzt und die Reaktionslösung mehrfach mit EA extrahiert. Die vereinten EA-Extrakte wurden getrocknet, filtriert und zur trockene eingeengt. Der erhaltene Rückstand wurde über Kieselgel mit CH₂Cl₂/Methanol als Laufmittel gereinigt, wobei 32 mg der Titelverbindung in Form eines amorphen Feststoffs gewonnen wurden.
Rₜ (Methode A) = 0.81 min MS (ES+) = 485 [M+H]⁺
¹H-NMR (500 MHz, DMSO-d₆): δ = 0.86 (m, 4H), 1.20-1.40 (m, 8 H), 1.63 (m, 2H), 1.70 (m, 2H), 1.95 (m, 1H), 2.90 (m, 1H), 3.45 (m, 1H), 3.52 (s, 3H), 3.70 (m, 1H), 3.94 (m, 1H), 4.13 (m, 2H), 4.52 (m, 1H), 5.68 (d, 1H), 6.30 (t, 1H), 6.55 (m, 1H), 7.00 (s, 1H), 7.13 (m, 1H), 7.72 (m, 2H).

### Beispiel 7

### 3-(6-Amino-pyridin-3-yl)-2-(1-cyclopentyl-1H-imidazol-4-yl)-propionsäure

### Beispiel 7a

### (1-Cyclopentyl-1H-imidazol-4-yl)-essigsäure-cyclopentylester

Imidazol-4-essigsäure Hydrochlorid (5,30 g; 32,60 mmol) und Cäsiumcarbonat (31,90 g, 97,80 mmol) wurde in absolutem DMF vorgelegt. Dazu wurde Cyclopentylbromid (10,5 ml; 97,80 mmol) gegeben. Es wurde 3 h bei 110 °C gerührt, dann über eine Klärschicht filtriert, der Rückstand mit CH₂Cl₂ gewaschen und das Filtrat unter vermindertem Druck eingeengt. Der Rückstand wurde in EA aufgenommen und die Lösung wurde mit Wasser und 0,5 N HCl gewaschen. Die organische Phase wurde über Na₂SO₄ getrocknet und das Lösemittel wurde dann unter vermindertem Druck entfernt. Nach Reinigung des Rohprodukts über eine Kartusche (70 g Silicagel) wurden 2,65 g (1-Cyclopentyl-1H-imidazol-4-yl)-essigsäure-cyclopentylester erhalten. Rₜ: (Methode B) = 0,89 min MS (ES+) = 263 [M+H]⁺

### Beispiel 7b

### 2-(1-Cyclopentyl-1H-imidazol-4-yl)-malonsäure-cyclopentylester-methylester

(1-Cyclopentyl-1H-imidazol-4-yl)-essigsäure-cyclopentylester (2,00 g; 7,60 mmol) wurde in Tetrahydrofuran (THF; 45 ml) gelöst. Nachdem die Reaktionslösung auf 0 °C gekühlt worden war, wurde Lithiumhexamethyldisilazan (20% in THF; 6,33 ml; 7,60 mmol) zugetropft. Es wurde für eine weitere Stunde bei 0 °C gerührt. Dann wurde Methylcyanoformiat (0,66 ml; 8,36 mmol) zugegeben. Es wurde 10 min bei 0 °C und dann 7 h bei RT gerührt. Die Reaktionslösung wurde dann auf gesättigte NH₄Cl-Lösung gegeben. Es wurde mit EA extrahiert, die organische Phase mit H₂O gewaschen und über Na₂SO₄ getrocknet. Das Lösemittel wurde unter vermindertem Druck entfernt. Nach Reinigung des Rohprodukts mittels Säulenchromatographie (120 g Silicagel; EtOAc/n-Heptan - 2/1) wurden 0,46 g 2-(1-Cyclopentyl-1H-imidazol-4-yl)-malonsäure-cydopentylester-methylester erhalten.
Rₜ (Methode B) = 1,01 min MS (ES+) = 321 [M+H]⁺

### Beispiel 7c

### 2-(6-Amino-pyridin-3-ylmethyl)-2-(1-cyclopentyl-1H-imidazol-4-yl)-malonsäure-cyclopentylester-methylester

2-(1-Cyclopentyl-1H-imidazol-4-yl)-malonsäure-cyclopentylester-methylester (0,46 g; 1,44 mmol) wurde in absolutem DMF gelöst (5 ml). Bei 0 °C wurde Natriumhydrid (50%; 0,07 g; 1,44 mmol) zugegeben. Es wurde 1 h bei RT gerührt und dann wieder auf 0 °C gekühlt. Dann wurde (5-Bromomethyl-pyridin-2-yl)- tert-butyl-carbamat (0,41 g; 1,44 mmol) zugegeben und 2 h bei RT gerührt. Unter Eiskühlung wurde mit H₂O gequencht und danach mit zweimal mit EA extrahiert. Die organische Phase wurde abgetrennt, über Na₂SO₄ getrocknet und das Lösemittel unter vermindertem Druck entfernt. Nach Reinigung des Rohprodukts über eine Kartusche (50 g Silicagel) wurden 0,31 g 2-(6-Amino-pyridin-3-ylmethyl)-2-(1-cyclopentyl-1H-imidazol-4-yl)-malonsäure-cyclopentylester-methylester erhalten.
Rₜ (Methode C) = 1,47 min MS (ES+) = 527 [M+H]⁺

### Beispiel 7d

### 3-(6-Amino-pyridin-3-yl)-2-(1-cyclopentyl-1H-imidazol-4-yl)-propionsäure

2-(6-Amino-pyridin-3-ylmethyl)-2-(1-cyclopentyl-1H-imidazol-4-yl)-malonsäure-cyclopentylester-methylester (0,30 g; 0,57 mmol) wurde in absolutem Ethanol (5 ml) gelöst. Bei 0 °C wurde ethanolische HCl zugegeben. Die Reaktionslösung wurde 48 h bei 10 °C stehengelassen. Dann wurde unter vermindertem Druck eingeengt und der Rückstand in 2N HCl aufgenommen. Es wurde in der Mikrowelle (3 x 4 min; 180 °C) erhitzt. Danach wurde mit EA gewaschen und die wässrige Phase mit 1 N NaOH neutralisiert und danach gefriergetrocknet. Der Rückstand wurde in Methanol aufgenommen und zweimal über eine Kartusche (C18) filtriert. Nach Einengen des Filtrats unter vermindertem Druck wurden 0,10 g 3-(6-Amino-pyridin-3-yl)-2-(1-cyclopentyl-1H-imidazol-4-yl)-propionsäure erhalten.
Rₜ (Methode C) = 0,64 min MS (ES+) = 301 [M+H]⁺
¹H-NMR (d⁶-DMSO): 1,60 (m, 2H); 1,73 (m, 4H); 2,18 (m, 2H), 2,87 (ddd, 1H); 3,58 (t, 1H); 3,78 (qt, 2H); 4,45 (t, 1H); 5,52 (s, 2H); 6,29 (d, 1H); 6,95 (s, 1H); 7,11 (dd; 1H); 7,55 (s, 1H); 7,658 (s, 1H)

Die folgenden Verbindungen wurden analog zu Beispiel 1 hergestellt:

### Beispiel 33

3-(6-Amino-pyridin-3-yl)-2-[1-(4,4-dimethyl-cyclohexyl)-1H-imidazol-4-yl]-propionsäure als Bishydrochlorid
Rt (Methode C) = 0.93 min. MS (ES+) = 343 [M+H]+
1H-NMR (500 MHz, d6-DMSO): δ = 0.93 (s, 3H); 1.00 (s, 3H); 1.33 (m, 2H); 1.48 (m, 2H); 1.85 (m, 4H); 3.04 (dd, 1H); 3.20 (dd, 1H); 4.11 (t, 1H); 4.16 (m, 1H); 6.90 (d, 1H); 7.73 (d, 2H); 7.81 (s, 1H); 8.02 (s, 2H, br); 9.14 (s, 1H).

### Beispiel 47

3-(6-Amino-pyridin-3-yl)-2-(1-cyclohexyl-1H-imidazol-4-yl)-propionsäureethylester Die Titelverbindung wurde als Hydrochlorid analog zu Beispiel 2 synthetisiert.
Rₜ (Methode C) = 0,87 min MS (ES+) = 343 [M+H]⁺
¹H-NMR (500 MHz, d⁶-DMSO): 1,12 (t, 3H), 1,13-1,27 (m, 1H), 1,31-1,41 (m, 2H), 1,61-1,73 (m, 3H), 1,80-1,86 (m, 2H), 1,98-2,05 (m, 2H), 3,12 (dd, 1H), 3,18 (dd, 1H), 4,12 (m, 2H) 4,21-4,27 (m, 2H), 6,93 (d, 1H), 7,72-7,78 (m, 3H), 8,08 (s, 2H), 9,12 (s, 1H).

### Pharmakologische Beispiele

Die hergestellten Substanzen wurden mit dem Actichrome Plasma TAFI Activity Kit der Firma American Diagnostica (Pr.Nr. 874) auf Inhibition von TAFla geprüft. Dabei wurden zu 1 µl 5 mM DMSO-Lösung der Substanz 29 µL Testpuffer (20mM Hepes, 150mM NaCl, pH 7,4) und 10 µL TAFla (American Diagnostica Pr.Nr. 874TAFIA; 2.5 /ml) gegeben und 15 Minuten bei Raumtemperatur in einer 96 half-well Mikrotiterplatte inkubiert. Die Enzymreaktion wurde durch Zugabe von 10 µL TAFIa "Developer" (1:2 mit Wasser vorverdünnt) gestartet. Der Zeitverlauf der Reaktion wurde bei 420 nm in einem Mikrotiterplattenreader (SpectraMax plus 384; Fa. Molecular Devices) über 15 Minuten verfolgt.

Die IC₅₀ wurde aus den gemittelten Werten (Doppelbestimmung) einer Verdünnungsreihe der Substanz mit Hilfe der Software Grafit 4 (Erithacus Software, UK) berechnet.
Tabelle 1 zeigt die Ergebnisse.

**Tabelle 1:**

| Verbindung aus | TAFla-Enzym-Assay IC₅₀ [□M] |
|---|---|
| Beispiel 1 | 0,030 |
| Beispiel 7 | 0,016 |
| Beispiel 33 | 0,003 |

## Patentansprüche

1. Verbindung der Formel la, 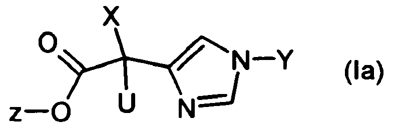 und/oder alle stereoisomeren Formen der Verbindung der Formel la und/oder Gemische dieser Formen in jedem Verhältnis, und/oder ein physiologisch verträgliches Salz der Verbindung der Formel Ia, wobei
U für Wasserstoffatom steht,
X für den Rest der Formel II
-(A1)ₘ-A2 (II)
steht, worin
m die ganze Zahl 1 bedeutet,
A1 für -CH₂- steht,
A2 für Aminopyridyl steht, worin Aminopyridyl unsubstituiert oder unabhängig voneinander ein-, zwei- oder dreifach durch Halogen oder -CH₃ substituiert ist,
Y für -(C₃-C₈)-Cycloalkyl steht, worin Cycloalkyl unsubstituiert oder unabhängig voneinander ein-, zwei- oder dreifach durch R1 substituiert ist, wobei R1 für
a) Phenyl, wobei Phenyl unsubstituiert oder ein-, zwei- oder dreifach unabhängig voneinander durch -(C₁-C₄)-Alkyl substituiert ist,
b) Triazolyl oder Pyridinyl,
c) -(C₁-C₄)-Alkyl,
d) -(C₃-C₆)-Cycloalkyl,
e) -CF₃,
f) -O-CF₃,
g) Fluor oder
h) Chlor steht, und
Z für 1) Wasserstoffatom,
2) -(C₁-C₆)-Alkyl,
3) -(C₁-C₆)-Alkyl-OH,
4) -(C₀-C₄)-Alkyl-(C₃-C₆)-Cycloalkyl oder
5) -(C₁-C₁₀)-Alkyl-O-C(O)-O-(C₃-C₆)-Cycloalkyl steht.

2. Verbindung der Formel la gemäß Anspruch 1, wobei
U für Wasserstoffatom steht,
X für den Rest der Formel II steht, worin
m die ganze Zahl 1 bedeutet,
A1 für -CH₂- steht,
A2 für den Rest 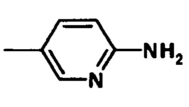 steht, welcher unsubstituiert oder unabhängig voneinander ein-, zwei- oder dreifach durch F, Cl, Br, J oder -CH₃ substituiert ist,
Y für -(C₃-C₈)-Cycloalkyl steht, worin Cycloalkyl unsubstituiert oder
unabhängig voneinander ein-, zwei- oder dreifach durch R1 substituiert ist, wobei R1 für
a) Phenyl, wobei Phenyl unsubstituiert oder ein-, zwei- oder dreifach unabhängig voneinander durch -(C₁-C₄)-Alkyl substituiert ist,
b) Pyridyl oder Tetrazolyl,
c) -(C₁-C₄)-Alkyl,
d) -(C₃-C₆)-Cycloalkyl,
e) -CF₃,
f) -O-CF₃,
g) Fluor oder
h) Chlor steht, und
Z für Wasserstoffatom steht.

3. Verbindung der Formel la gemäß der Ansprüche 1 oder 2, **dadurch gekennzeichnet, dass** es die Verbindung
3-(6-Amino-pyridin-3-yl)-2-(1-cyclohexyl-1H-imidazol-4-yl)-propionsäure,
3-(6-Amino-pyridin-3-yl)-2-(1-cyclohexyl-1H-imidazol-4-yl)-propionsäuremethylester.
3-(6-Amino-pyridin-3-yl)-2-(1-cyclohexyl-1H-imidazol-4-yl)-propionsäureisopropylester,
3-(6-Amino-pyridin-3-yl)-2-(1-cyclohexyl-1H-imidazol-4-yl)-propionsäurecyclopropyl-methylester,
3-(6-Amino-pyridin-3-yl)-2-(1-cyclohexyl-1H-imidazol-4-yl)-propionsäure-2-hydroxy-ethylester,
3-(6-Amino-pyridin-3-yl)-2-(1-cyclohexyl-1H-imidazol-4-yl)-propionsäure-1-cyclohexyl-oxycarbonyloxy-ethylester,
3-(6-Amino-pyridin-3-yl)-2-(1-cyclopentyl-1H-imidazol-4-yl)-propionsäure,
3-(6-Amino-pyridin-3-yl)-2-[1-(4,4-dimethyl-cyclohexyl)-1H-imidazol-4-yl]-propionsäure, oder
3-(6-Amino-pyridin-3-yl)-2-(1-cyclohexyl-1H-imidazol-4-yl)-propionsäureethylester ist.

4. Verfahren zur Herstellung der Verbindung der Formel la gemäß einem oder mehreren der Ansprüche 1 bis 3, **dadurch gekennzeichnet, dass** man
a) eine Verbindung der Formel VII 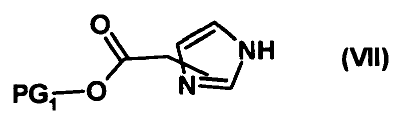 wobei PG1 für eine Carboxylschutzgruppe steht, in eine Verbindung der Formel la gemäß Anspruch 1 umwandelt,
b) eine nach Verfahren a) hergestellte Verbindung der Formel la, oder eine geeignete Vorstufe der Formel la, die aufgrund ihrer chemischen Struktur in enantio-meren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
c) die nach den Verfahren a) oder b) hergestellte Verbindung der Formel la entweder in freier Form isoliert oder im Falle des Vorliegens von sauren oder basischen Gruppen in physiologisch verträgliche Salze umwandelt.

5. Arzneimittel, **gekennzeichnet durch** einen wirksamen Gehalt an mindestens einer Verbindung der Formel la gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

6. Verwendung der Verbindung der Formel la gemäß einem oder mehreren der Ansprüche 1 bis 3 zur Herstellung eines Arzneimittels zur Prophylaxe und Therapie all solcher Erkrankungen, die die mit Thrombosen, Embolien, Hyperkoagulabilität oder fibrotischen Veränderungen einhergehen.

7. Verwendung gemäß Anspruch 6, **dadurch gekennzeichnet, dass** es sich um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder um die disseminierte intravaskuläre Koagulation, Sepsis und anderen intravaskulären Ereignissen, die mit einer Entzündung einhergehen, Atherosklerose, Diabetes und dem metabolischen Syndrom und dessen Folgen, Tumorwachstum und Tumormetastasierung, Störungen des hämostatischen Systems wie Fibrinablagerungen, fibrotische Veränderungen der Lunge wie die chronische obstruktive Lungenerkrankung, das adult respiratory distress syndrome oder Fibrinablagerungen des Auges nach Augenoperationen oder Verhinderung und/oder Behandlung von Narbenbildung.

## Claims

1. A compound of the formula la 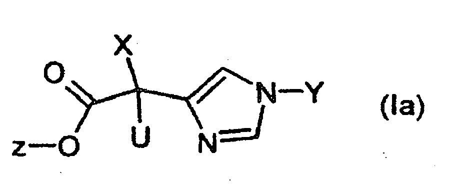 and/or all stereoisomeric forms of the compound of the formula la and/or mixtures of these forms in any ratio, and/or a physiologically tolerated salt of the compound of the formula la, where
U is hydrogen atom,
X is the radical of the formula II
-(A1)ₘ-A2 (II)
in which
m is the integer 1,
A1 is -CH₂-
A2 is aminopyridyl, in which aminopyridyl is unsubstituted or substituted independently of one another once, twice or three times by halogen or -CH₃,
Y is -(C₃-C₈)-cycloalkyl, in which cycloalkyl is unsubstituted or substituted independently of one another once, twice or three times by R1, where R1 is
a) phenyl, where phenyl is unsubstituted or substituted once, twice or three times independently of one another by -(C₁-C₄) alkyl,
b) triazolyl or pyridinyl,
c) -(C₁-C₄)-alkyl,
d) -(C₃-C₆)-cycloalkyl,
e) -CF₃,
f) -O-CF₃,
g) fluorine or
h) chlorine, and
Z is 1) hydrogen atom,
2) -(C₁-C₆)-alkyl,
3) -(C₁-C₆)-alkyl-OH,
4) -(C₀-C₄)-alkyl-(C₃-C₆)-cycloalkyl or
5) -(C₁-C₁₀)-alkyl-O-C(O)-O-(C₃-C₆)-cycloalkyl.

2. The compound of the formula la as claimed in claim 1, where
U is hydrogen atom,
X is the radical of the formula II in which
m is the integer 1,
A1 is -CH₂-,
A2 is the radical 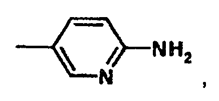 which is unsubstituted or substituted independently of one another once, twice or three times by F, Cl, Br, I or -CH₃,
Y is -(C₃-C₈)-cycloalkyl, in which cycloalkyl is unsubstituted or substituted independently of one another once, twice or three times by R1, where R1 is
a) phenyl, where phenyl is unsubstituted or substituted once, twice or three times independently of one another by -(C₁-C₄)-alkyl,
b) pyridyl or tetrazolyl,
c) -(C₁-C₄)-alkyl,
d) -(C₃-C₆)-cycloalkyl,
e) -CF₃,
f) -O-CF₃,
g) fluorine or
h) chlorine, and
Z is hydrogen atom.

3. The compound of the formula Ia as claimed in claims 1 or 2, which is the compound
3-(6-amino-pyridin-3-yl)-2-(1-cyclohexyl-1H-imidazol-4-yl) propionic acid,
methyl 3-(6-amino-pyridin-3-yl)-2-(1-cyclohexyl-1H-imidazol-4-yl) propionate,
isopropyl 3-(6-amino-pyridin-3-yl)-2-(1-cyclohexyl-1H-imidazol-4-yl) propionate,
cyclopropylmethyl 3-(6-amino-pyridin-3-yl)-2-(1-cyclohexyl-1H-imidazol-4-yl) propionate,
2-hydroxyethyl 3-(6-amino-pyridin-3-yl)-2-(1-cyclohexyl-1H-imidazol-4-yl) propionate,
1-cyclohexyloxycarbonyloxyethyl 3-(6-amino-pyridin-3-yl)-2-(1-cyclohexyl-1H-imidazol-4-yl) propionate,
3-(6-amino-pyridin-3-yl)-2-(1-cyclopentyl-1H-imidazol-4-yl) propionic acid,
3-(6-amino-pyridin-3-yl)-2-[1-(4, 4-dimethyl-cyclohexyl)-1H-imidazol-4-yl] propionic acid, or
ethyl 3-(6-amino-pyridin-3-yl)-2-(1-cyclohexyl-1H-imidazol-4-yl) propionate.

4. A process for preparing the compound of the formula la as claimed in one or more of claims 1 to 3, which comprises
a) a compound of the formula VII 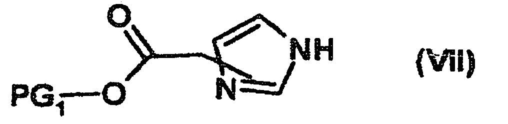 where PG1 is a carboxyl protective group, being converted into a compound of the formula la as claimed in claim 1,
b) a compound of the formula la which has been prepared by process
a) or a suitable precursor of the formula la which occurs owing to its chemical structure in enantiomeric forms being fractionated by salt formation with enantiopure acids or bases, chromatography on chiral stationary phases or derivatization using chiral enantiopure compounds such as amino acids, separation of the diastereomers obtained in this way, and elimination of the chiral auxiliary groups into the pure enantiomers, or
c) the compound of the formula la prepared by processes a) or b) being either isolated in free form or, in the case where acidic or basic groups are present, converted into physiologically tolerated salts.

5. A medicament having an effective content of at least one compound of the formula la as claimed in one or more of claims 1 to 3 together with a pharmaceutically suitable and physiologically tolerated carrier, additive and/or other active ingredients and excipients.

6. The use of the compound of the formula la as claimed in one or more of claims 1 to 3 for producing a medicament for the prophylaxis and therapy of all disorders associated with thromboses, embolisms, hypercoagulability or fibrotic changes.

7. The use as claimed in claim 6, which is applied to myocardial infarction, angina pectoris and other types of acute coronary syndrome, stroke, peripheral vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations, or reduction of the risk of thrombosis following surgical procedures as in knee and hip joint operations, or disseminated intravascular coagulation, sepsis and other intravascular events which are associated with an inflammation, atherosclerosis, diabetes and the metabolic syndrome and its sequelae, tumor growth and tumor metastasis, impairments of the hemostatic system such as fibrin deposits, fibrotic changes of the lung such as chronic obstructive lung disease, adult respiratory distress syndrome or fibrin deposits in the eye after eye operations or prevention and/or treatment of scar formation.

## Revendications

1. Composé de la formule la 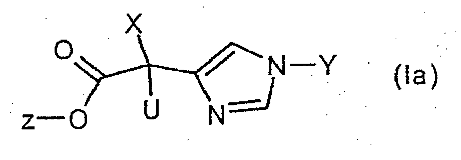 et/ou toutes les formes stéréoisomères du composé de la formule la et/ou les mélanges de ces formes dans des proportions quelconques et/ou un sel physiologiquement toléré du composé de la formule la, dans laquelle
U représente un atome d'hydrogène,
X représente le radical de la formule II
-(A1)ₘ,-A2 (II)
dans laquelle
m vaut le nombre entier 1,
A1 représente un radical -CH₂-
A2 représente un groupe aminopyridyle, dans lequel le groupe aminopyridyle est non substitué ou substitué indépendamment l'un de l'autre une fois, deux fois ou trois fois par un halogène ou un groupe -CH₃,
Y représente un groupe cycloalkyle (C₃ à C₈), dans lequel le groupe cycloalkyle est non substitué ou substitué indépendamment l'un de l'autre une fois, deux fois ou trois fois par R1amment l'un de l'autre une fois, deux fois ou trois fois par R1,
où R1 représente
a) un groupe phényle, dans lequel le groupe phényle est non substitué ou substitué indépendamment l'un de l'autre une fois, deux fois ou trois fois par un groupe alkyle (C₁ à C₄),
b) un groupe triazolyle ou un groupe pyridinyle,
c) un groupe alkyle (C₁ à C₄),
d) un groupe cycloalkyle (C₃ à C₆),
e) un groupe -CF₃,
f) un groupe -O-CF₃,
g) un atome de fluor ou
h) un atome de chlore,
et
Z représente 1) un atome d'hydrogène,
2) un groupe alkyle (C₁ à C₆),
3) un radical alkyl (C₁ à C₆)-OH,
4) un radical alkyl (C₀ à C₄)-cycloalkyle (C₃ à C₆), ou
5) un radical alkyl (C₁ à C₁₀)-O-C(O)-O-cycloalkyle (C₃ à C₆).

2. Composé de la formule la selon la revendication 1, dans laquelle
U représente un atome d'hydrogène,
X représente le radical de la formule II dans laquelle
m vaut le nombre entier 1,
A1 représente un radical -CH₂-,
A2 représente le radical 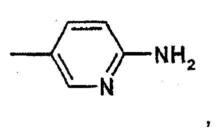 lequel est non substitué ou substitué indépendamment l'un de l'autre une fois, deux fois ou trois fois par un atome F, un atome Cl, un atome Br, un atome I ou un groupe -CH₃, Y représente un groupe cycloalkyle (C₃ à C₈), dans lequel le groupe cycloalkyle est non substitué ou substitué indépendamment l'un de l'autre une fois, deux fois ou trois fois par R1,
où R1 représente
a) un groupe phényle, dans lequel le groupe phényle est non substitué ou substitué indépendamment l'un de l'autre une fois, deux fois ou trois fois par un groupe alkyle (C₁ à C₄),
b) un groupe pyridyle ou un groupe tétrazolyle,
c) un groupe alkyle (C₁ à C₄),
d) un groupe cycloalkyle (C₃ à C₆),
e) un groupe -CF₃,
f) un groupe -O-CF₃,
g) un atome de fluor ou
h) un atome de chlore,
et
Z représente un atome d'hydrogène.

3. Composé de la formule la selon les revendications 1 ou 2, **caracterisé en ce qu'**il est le composé
l'acide 3-(6-aminopyridin-3-yl)-2-(1-cyclohexyl-1H-imidazol-4-yl) propionique,
le 3-(6-aminopyridin-3-yl)-2-(1-cyclohexyl-1H-imidazol-4-yl) propionate de méthyle,
le 3-(6-aminopyridin-3-yl)-2-(1-cyclohexyl-1H-imidazol-4-yl)- propionate d'isopropyle,
le 3-(6-aminopyridin-3-yl)-2-(1-cyclohexyl-1H-imidazol-4-yl) propionate de cyclopropylméthyle,
le 3-(6-aminopyridin-3-yl)-2-(1-cyclohexyl-1H-imidazol-4-yl) propionate de 2-hydroxyéthyle,
le 3-(6-aminopyridin-3-yl)-2-(1-cyclohexyl-1H-imidazol-4-yl) propionate de 1-cyclohexyloxycarbonyloxyéthyle,
l'acide 3-(6-aminopyridin-3-yl)-2-(1-cyclopenty)-1H-imidazol-4-yl) propionique,
l'acide 3-(6-aminopyridin-3-yl)-2-[1-(4,4-diméthylcyclohexyl)-1H-imidazol-4-yl] propionique,
ou
le 3-(6-aminopyridin-3-yl)-2-(1-cyclohexyl-1H-imidazol-4-yl) propionate d'éthyle.

4. Procédé de préparation du composé de la formule la selon l'une ou plusieurs des revendications 1 à 3, **caracterisé en ce que**
a) un composé de la formule VII 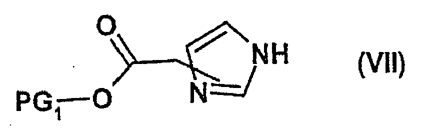 dans laquelle PG1 représente un groupe protecteur du groupe carboxyle, est converti en un composé de la formule la selon la revendication 1,
b) un composé de la formule la qui a été préparé par le procédé a) ou un précurseur approprié de la formule la qui est obtenu en raison de sa structure chimique dans des formes énantiomériques est fractionné par une formation de sel avec des acides ou des bases énantiopurs, par chromatographie en phases chirales stationnaires ou par dérivatisation en utilisant des composés chiraux énantiopurs tels que des acides aminés, par séparation des diastéréoisomères obtenus de cette manière et par élimination des groupes chiraux auxiliaires pour obtenir des énantiomères purs, ou
c) le composé de la formule la préparé par le procédé a) ou b) est soit isolé sous sa forme libre soit, dans le cas où des groupes acides ou basiques sont présents, converti en des sels physiologiquement tolérés.

5. Médicament **caracterisé par** une teneur efficace en au moins un composé de la formule la selon l'une ou plusieurs des revendications 1 à 3 conjointement avec un support ou un additif pharmaceutiquement approprié et physiologiquement toléré, et/ou d'autres ingrédients actifs et excipients.

6. Utilisation du composé de la formule la selon l'une ou plusieurs des revendications 1 à 3 pour produire un médicament pour la prophylaxie et la thérapie de tous les troubles associés aux thromboses, aux embolies, à l'hypercoagulabilité ou aux changements fibreux.

7. Utilisation selon la revendication 6, **caracterisé en ce qu'**il s'applique à l'infarctus du myocarde, à l'angine de poitrine et aux autres types de syndrome coronarien aigu, à l'arrêt vasculaire cérébral, aux troubles vasculaires périphériques, à la thrombose veineuse profonde, à l'embolie pulmonaire, aux évènements emboliques ou thrombotiques causés par une arythmie du coeur, aux évènements cardiovasculaires tels qu'une resténose suite à une revascularisation et à l'angioplastie et à des procédures semblables telles que des implants d'endoprothèses vasculaires et des pontages, ou à la réduction du risque d'une thrombose suite à des procédures chirurgicales telles que des opérations du genou et de l'articulation de la hanche, ou à une coagulation intravasculaire disséminée, à une sepsie et à d'autres évènements intravasculaires qui sont associés à une inflammation, à une athérosclérose, au diabète et au syndrome métabolique et ses séquelles, à une croissance tumorale et à une métastase tumorale, aux troubles du système hémostatique tels que des dépôts fibreux, à des changements fibreux des poumons tels qu'une bronchopneumopathie chronique obstructive, un syndrome de détresse respiratoire chez l'adulte, ou des dépôts fibreux dans l'oeil après des opérations des yeux ou à la prévention et/ou au traitement de la formation de cicatrices.
